# FASCICULE DE BREVET EUROPEEN

(11) **EP 2 488 128 B1**
(45) Date de publication et mention de la délivrance du brevet: **04.09.2013**
(21) Numéro de dépôt: 10785518.1
(22) Date de dépôt: 12.10.2010
(51) Int. Cl.: A61F 2/32, A61F 2/34

(54) **COTYLE PROTHETIQUE A GRANDE CAPACITE DE RETENTION**
HÜFTGELENKPFANNE MIT HOHER RETENTIONSKAPAZITÄT
ACETABULAR CUP WITH HIGH RETENTION CAPACITY

(30) Priorité: 12.10.2009 FR 0957130
(43) Date de publication de la demande: 22.08.2012
(73) Titulaire: Gradel, Thomas, 74970 Marignier (FR)
(72) Inventeur: Gradel, Thomas, 74970 Marignier (FR)
(74) Mandataire: Poncet, Jean-François
(86) Numéro de dépôt international: PCT/IB2010/054603
(87) Numéro de publication internationale: WO 2011/045737

(56) Documents cités:
- WO-A1-88/07845
- DE-A1- 4 102 510
- FR-A1- 2 876 278
- US-A1- 2005 060 040

## Description

### DOMAINE TECHNIQUE DE L'INVENTION

La présente invention concerne un cotyle prothétique destiné à remplacer le cotyle naturel de la hanche.

Une prothèse totale de hanche comprend deux parties constituant une articulation à rotule, à savoir une première partie destinée à être implantée sur le bassin d'un patient et une seconde partie destinée à être implantée sur le fémur.

La première partie de la prothèse comporte généralement une tige destinée à pénétrer dans le canal médullaire du fémur, et dont l'extrémité proximale se raccorde par un col à une tête sphérique destinée à pénétrer dans la partie femelle de l'articulation.

La seconde partie de la prothèse, qui doit être implantée dans le bassin, et que l'on désignera globalement par l'appellation cotyle, comprend habituellement une cupule d'insertion hémisphérique, que l'on vient loger dans une cavité cotyloïdienne préparée de l'os du bassin, et dans laquelle on loge un insert articulaire conformé pour recevoir la tête sphérique. De façon courante, la cupule d'insertion est métallique. L'insert articulaire est en une matière à faible coefficient de frottement telle que le polyéthylène ou une céramique.

On peut distinguer, dans les dispositifs connus, les cotyles à simple mobilité, les cotyles à double mobilité, et les cotyles à cupule mobile.

Dans les cotyles à simple mobilité, l'insert en polyéthylène ou en céramique est fixe dans une cupule d'insertion, et comporte une cavité articulaire sensiblement hémisphérique coaxiale qui permet l'engagement et le pivotement de la tête sphérique de la première partie de prothèse.

Les mouvements de rotation de l'articulation se font alors entre la tête sphérique de la première partie de prothèse et la cavité articulaire de l'insert articulaire.

Dans un cotyle à double mobilité, l'insert articulaire est lui-même monté rotatif dans la cupule d'insertion, réalisant ainsi une première surface de glissement entre la cupule d'insertion et l'insert articulaire, et une seconde surface de glissement entre l'insert articulaire et la tête sphérique.

Dans les cotyles à cupule mobile, l'insert articulaire en céramique présente une surface externe sphérique pour être monté rotatif directement dans la cavité cotyloïdienne du bassin d'un patient. En variante, un insert articulaire en polyéthylène est engagé fixe dans une cupule métallique comportant une surface externe sphérique elle-même montée rotative dans la cavité cotyloïdienne du bassin d'un patient.

Le principal problème, lors de l'utilisation d'une prothèse de hanche, est le risque de luxation. La luxation est une sortie de la tête fémorale sphérique hors de la cavité articulaire.

Pour réduire les risques de luxation, on a déjà proposé différents moyens.

Par exemple, les cotyles à double mobilité ont pour effet de réduire quelque peu le risque de luxation. Cette réduction est toutefois insuffisante.

Un moyen supplémentaire pour réduire les risques de luxation a été proposé sous forme d'une augmentation de la profondeur de la cavité articulaire de l'insert articulaire, de façon que la tête soit engagée selon une calotte sphérique légèrement plus grande que l'hémisphère. En pratique, il faut alors engager la tête en force dans la cavité articulaire, et cela nécessite un outil spécifique de type presse.

Jusqu'à présent, les têtes sphériques de la partie mâle d'articulation ont constitué un élément interchangeable, rapporté à l'extrémité du col, qui permet au praticien, lors de la pose de la prothèse, d'adapter aisément le diamètre de la tête et la longueur du col par un simple choix de la tête appropriée prise dans une série de têtes ayant des diamètres différents et des logements à profondeurs différentes pour l'engagement de l'extrémité de col. Le choix de la tête est nécessairement effectué par le praticien, en fonction des particularités anatomiques du patient qui reçoit la prothèse.

Il en résulte que c'est le praticien qui doit ensuite engager en force la tête dans la cavité articulaire du cotyle articulaire, au moyen de l'outil de type presse. Cette opération d'engagement en force présente des risques importants de dégradation de la surface externe de la tête et de la surface interne de l'insert articulaire, de sorte que les surfaces articulaires se trouvent alors dégradées, réduisant la durée de vie de la prothèse.

En outre, s'agissant d'une opération effectuée par le praticien au bloc opératoire, avec un outil de type presse relativement sommaire, la force admissible pour l'engagement de la tête dans la cavité du cotyle articulaire est nécessairement limitée, ce qui limite simultanément la capacité de rétention de la tête dans l'insert articulaire. Il y a donc encore un besoin pour augmenter cette capacité de rétention.

Le document DE 41 02 510 A1 enseigne d'augmenter la force de rétention de la tête dans l'insert articulaire en prévoyant un moyen de rétention constitué par un prolongement du cotyle qui augmente au-delà de 180° l'angle de sa cavité, sur une portion de sa périphérie. Cependant, le document ne décrit pas de solution à la difficulté d'introduction de tête qui en résulte.

En alternative, on a proposé d'augmenter la force de rétention de la tête dans l'insert articulaire en adaptant une bague de rétention dans une gorge prévue à l'entrée de la cavité de l'insert articulaire. La bague est en polyéthylène. Elle peut être fendue, ou présenter des moyens d'encliquetage comme décrit dans le document WO 88/07845 A1. Grâce à sa fente transversale ou à ses moyens d'encliquetage, et au fait que le polyéthylène est un matériau relativement élastique, la bague peut être aisément déformée en réduisant son diamètre pour l'introduire à l'entrée de la cavité, puis relâchée pour qu'elle reprenne son diamètre initial et s'engage par sa périphérie dans la gorge correspondante de l'insert articulaire. La bague s'oppose alors à l'extraction de la tête hors de l'insert articulaire. Mais il reste un risque d'éjection de la bague de rétention elle-même lors d'un effort d'extraction de la tête hors de l'insert articulaire, du fait de la souplesse de la bague résultant de la présence de la fente transversale. Ainsi, la capacité de rétention est encore insuffisante.

### EXPOSE DE L'INVENTION

L'invention résulte de l'observation selon laquelle tous les moyens laissés à disposition du praticien pour adapter la tête dans l'insert articulaire au bloc opératoire ne permettent pas l'obtention d'une capacité de rétention suffisante, et il semble que la stérilisation a une influence négative sur cette capacité de rétention.

Le problème proposé par la présente invention est de réduire sensiblement le risque de luxation d'une prothèse de hanche, en conférant au cotyle une capacité de rétention nettement augmentée, de nature à écarter tout risque de luxation, en évitant l'influence négative de la stérilisation sur cette capacité de rétention.

Ainsi, pour atteindre ces buts ainsi que d'autres, l'invention propose un cotyle prothétique de hanche comprenant :
- un insert articulaire, comportant une face réceptrice dans laquelle est ouverte une cavité à surface articulaire concave sensiblement hémisphérique,
- une tête sphérique apte à être engagée dans la cavité articulaire de l'insert articulaire,
- un moyen de rétention pour s'opposer à la sortie axiale de la tête sphérique hors de la cavité articulaire de l'insert articulaire lorsque celle-ci est engagée dans la cavité articulaire,
   dans lequel :

- l'ensemble insert articulaire-tête sphérique est conditionné stérile dans une enveloppe protectrice,
- dans l'enveloppe protectrice, la tête sphérique est engagée dans la cavité articulaire de l'insert articulaire et est retenue par le moyen de rétention.

Cette disposition permet d'assembler le cotyle lors de la fabrication en usine et de réaliser l'étape de stérilisation après l'étape d'assemblage de la tête sphérique dans l'insert articulaire. On a constaté, selon la présente invention, que l'étape d'assemblage, par engagement en force d'un élément dans un autre, réduit les propriétés mécaniques de l'un au moins des deux éléments lorsque celui-ci est en polyéthylène ou autre matière plastique équivalente et a été préalablement soumis à une étape de stérilisation. Ainsi, le fait de réaliser la stérilisation après assemblage évite la dégradation des propriétés mécaniques de l'élément, et augmente la capacité de rétention de la tête dans l'insert articulaire.

L'effet d'augmentation de la capacité de rétention a pu être mis en évidence lors d'essais dont les résultats sont exposés dans la description qui suit.

En pratique, grâce à ces dispositions, la force de rétention de la tête sphérique exercée par le moyen de rétention peut être supérieure à un seuil de force déterminé, ce seuil de force étant nettement supérieur à la force de rétention obtenue par les dispositifs actuellement connus, pour des matériaux donnés et des géométries données des éléments formant la prothèse.

Selon un premier mode de réalisation, dans la cavité articulaire et le moyen de rétention, la tête sphérique est engagée selon une calotte sphérique plus grande que l'hémisphère.

La calotte sphérique peut avantageusement s'étendre selon un angle supérieur à 190°.

De la sorte, le moyen de rétention peut être constitué par l'insert articulaire lui-même, réalisé en polyéthylène ou autre matière plastique équivalente, et comportant un tronçon de rétention en contre-dépouille de surface articulaire.

Dans ce cas, l'insert articulaire peut aussi comporter une surface externe en calotte sphérique pour être montée rotatif dans une cupule d'insertion et pour constituer ainsi un cotyle à double mobilité.

Selon un autre mode de réalisation, le moyen de rétention comprend une bague annulaire continue, comportant une surface de retenue en couronne sphérique apte à s'engager fonctionnellement avec jeu contre la tête sphérique, et comportant des excroissances périphériques de fixation aptes à s'imbriquer fonctionnellement avec des retraits correspondants de l'insert articulaire ou avec des retraits correspondants d'une cupule périphérique entourant l'insert articulaire, pour interdire l'extraction de la bague annulaire continue.

L'absence de fente transversale dans la bague annulaire continue confère à la bague une grande raideur qui évite le risque d'extraction de la bague elle-même lors d'une poussée d'extraction de la tête sphérique hors de l'insert articulaire.

Dans ce cas, la bague annulaire continue peut avantageusement être en polyéthylène ou en une autre matière plastique équivalente.

Du fait du caractère continu de la bague annulaire constituant le moyen de rétention du second mode de réalisation, et du fait de l'angle supérieur à 190° de la calotte sphérique selon laquelle la tête est engagée dans la cavité articulaire et le moyen de rétention, on réalise une grande capacité de rétention de la tête sphérique dans l'insert articulaire.

Selon l'invention, cette capacité de rétention est encore augmentée en prévoyant un procédé d'assemblage pour réaliser un cotyle prothétique de hanche selon l'invention, avec une tête sphérique engagée dans une cavité d'un insert articulaire et retenue par un moyen de rétention, le procédé ayant une étape de stérilisation des composants du cotyle et une étape d'assemblage de la tête sphérique dans l'insert articulaire et le moyen de rétention ; selon le procédé, on réalise l'étape de stérilisation après l'étape d'assemblage.

Pour réduire ou éviter encore la détérioration des propriétés mécaniques des éléments à assembler lors de l'engagement de la tête dans l'insert articulaire et le moyen de rétention, au cours de l'étape d'assemblage, on peut avantageusement soumettre le moyen de rétention à une sollicitation thermique différentielle qui modifie temporairement ses dimensions pour faciliter l'assemblage, puis, après assemblage, on laisse le moyen de rétention reprendre la température ambiante pour qu'il s'oppose alors à l'extraction de la tête sphérique à l'écart de l'insert articulaire.

Selon un premier mode de réalisation, dans ce procédé :
- le moyen de rétention est l'insert articulaire lui-même, réalisé en polyéthylène ou autre matière plastique équivalente,
- la sollicitation thermique différentielle consiste à chauffer l'insert articulaire pour augmenter son diamètre intérieur et faciliter ainsi l'introduction axiale de la tête sphérique.

Selon un autre mode de réalisation, dans ce procédé :
- le moyen de rétention est une bague annulaire continue en polyéthylène ou autre matière plastique équivalente,
- la sollicitation thermique différentielle consiste à refroidir la bague annulaire continue pour diminuer son diamètre intérieur et faciliter ainsi l'engagement des excroissances périphériques de la bague dans les retraits correspondants de l'insert articulaire ou de la cupule périphérique.

### DESCRIPTION SOMMAIRE DES DESSINS

D'autres objets, caractéristiques et avantages de la présente invention ressortiront de la description suivante de modes de réalisation particuliers, faite en relation avec les figures jointes, parmi lesqueiles :
- les figures 1 à 3 illustrent, respectivement en perspective, en vue de face, et en coupe diamétrale de côté, un cotyle prothétique de hanche selon un premier mode de réalisation de la présente invention ;
- les figures 4 à 6 illustrent, respectivement en perspective, en vue de face, et en coupe diamétrale de côté, une tête sphérique telle que celle du mode de réalisation des figures 1 à 3 ;
- les figures 7 à 9 illustrent, respectivement en perspective, en vue de face, et en coupe diamétrale de côté, l'insert articulaire du cotyle prothétique des figures 1 à 3 ;
- les figures 10 à 12 illustrent, respectivement en perspective, en vue de face, et en coupe diamétrale de côté, l'ensemble insert articulaire-tête sphérique assemblé, tel que conditionné à l'état stérile dans une enveloppe protectrice pour sa distribution au site d'utilisation ;
- les figures 13 à 15 illustrent, respectivement en perspective, en vue de face, et en coupe diamétrale de côté, un cotyle prothétique de hanche selon un second mode de réalisation de la présente invention ;
- les figures 16 à 18 illustrent, respectivement en perspective, en vue de face, et en coupe diamétrale de côté, un cotyle prothétique de type cupule mobile selon un troisième mode de réalisation de la présente invention ;
- les figures 19 à 21 illustrent, respectivement en perspective, en vue de face, et en coupe diamétrale de côté, un cotyle prothétique de hanche de type cupule mobile selon un quatrième mode de réalisation de la présente invention ; et
- la figure 22 illustre en perspective une bague annulaire continue.

### DESCRIPTION DES MODES DE REALISATION PREFERES

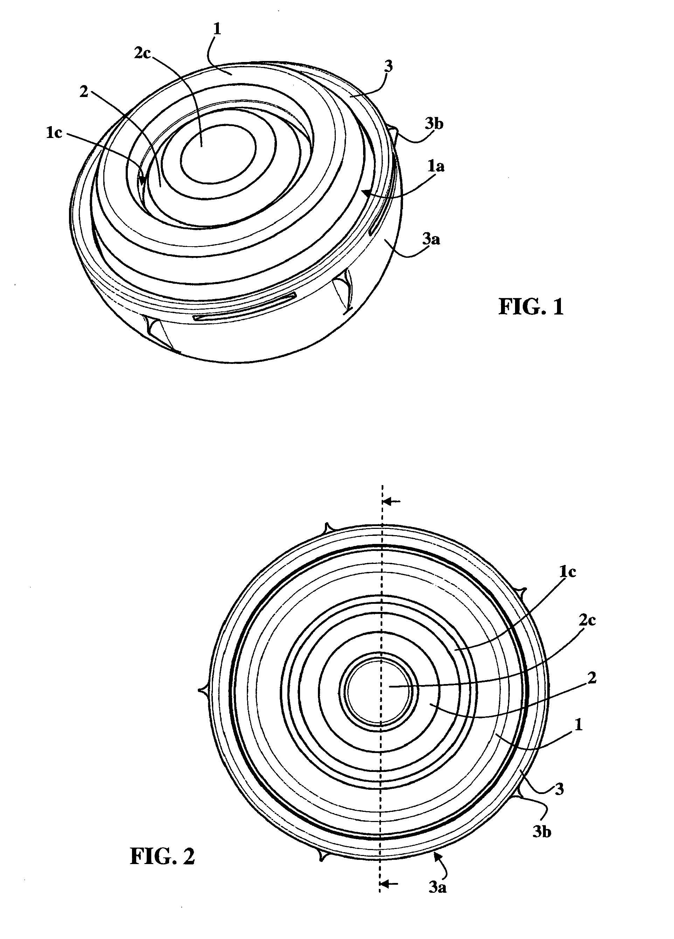

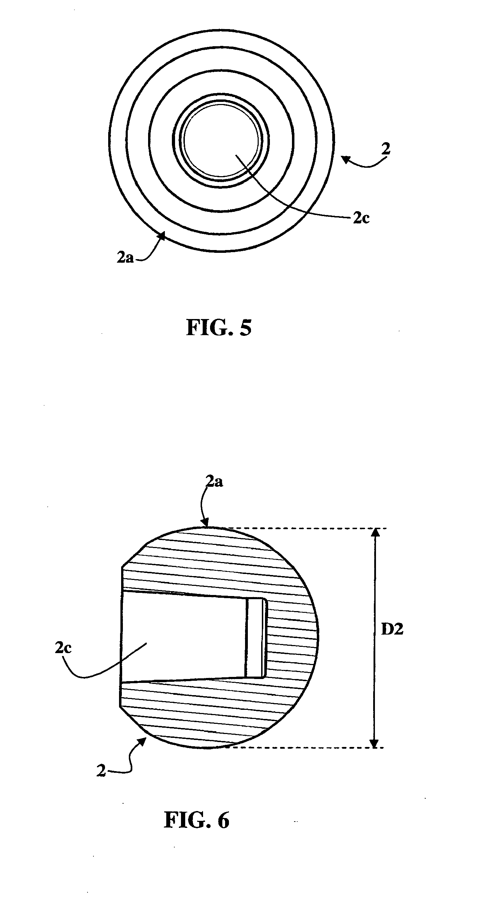

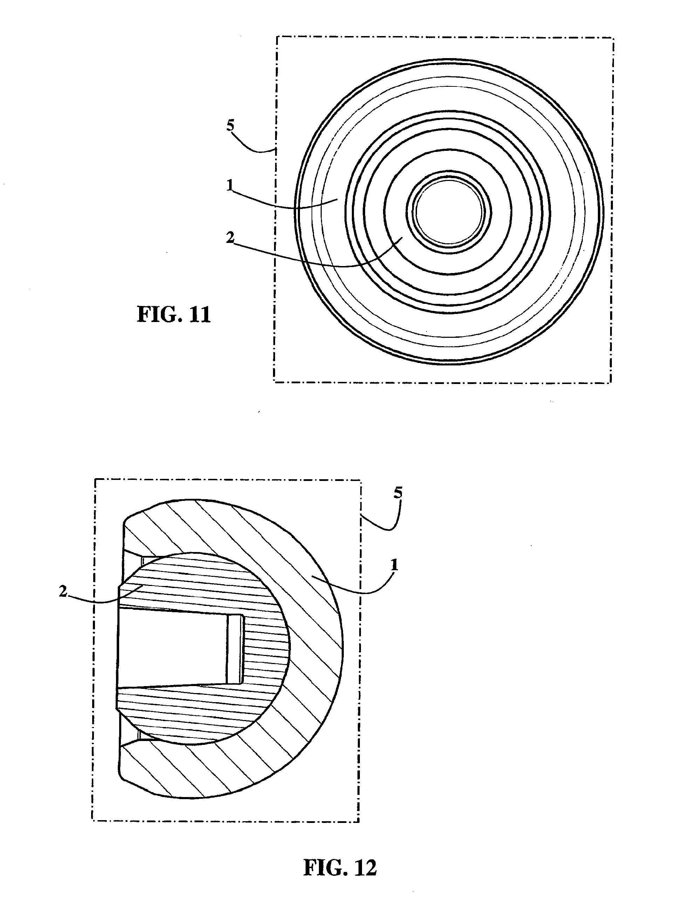

On considère les figures 1 à 12, qui illustrent la structure d'un cotyle prothétique à double mobilité selon un premier mode de réalisation de la présente invention.

Après assemblage complet, le cotyle prothétique est tel qu'illustré sur les figures 1 à 3. Il comprend un insert articulaire 1, une tête sphérique 2, et une cupule d'insertion 3.

La cupule d'insertion 3 est métallique. Elle présente une face extérieure d'ancrage 3a convexe, sensiblement hémisphérique, et conformée pour être ancrée dans une cavité cotyloïdienne du bassin d'un patient. Comme on le voit plus clairement sur les figures 1 et 2, la face extérieure d'ancrage comporte avantageusement des saillies 3b pour faciliter son ancrage dans la cavité cotyloïdienne du bassin. La cupule d'insertion 3 comporte une face intérieure de réception 3c, de forme sphérique, polie-miroir, pour constituer une surface d'articulation recevant l'insert articulaire 1. L'insert articulaire 1 est réalisé en polyéthylène ou autre matière plastique équivalente. Il comporte une face extérieure 1a sphérique s'engageant dans la face intérieure de réception 3c de la cupule d'insertion 3 pour permettre le pivotement de l'insert articulaire 1 dans la cupule d'insertion 3. L'insert articulaire 1 comporte une face réceptrice 1b dans laquelle est ouverte une cavité articulaire 1c à surface articulaire 1d concave sensiblement hémisphérique.

La tête sphérique 2 est engagée dans la cavité articulaire 1c de l'insert articulaire 1, dans laquelle elle est retenue par un moyen de rétention qui s'oppose à sa sortie axiale sous l'effet d'une force d'extraction illustrée par la flèche 4.

Dans ce mode de réalisation, le moyen de rétention est constitué par l'insert articulaire 1 lui-même, qui comporte pour cela une surface articulaire 1d s'étendant selon un angle A supérieur à 190°. Du fait de la valeur de l'angle A supérieure à 190°, la surface articulaire 1d comporte un tronçon 10a en contre-dépouille, qui va en se rétrécissant vers la face réceptrice 1b. Il en résulte que le diamètre D1 de l'ouverture de l'insert articulaire 1 est inférieur au diamètre D2 de la tête sphérique 2, qui ne peut donc sortir de la cavité articulaire 1c que sous l'effet d'une force d'extraction 4 supérieure à une valeur élevée.

La tête sphérique 2 comporte une surface articulaire extérieure 2a sphérique, qui se trouve ainsi engagée dans la cavité articulaire 1c selon une calotte sphérique 2b plus grande que l'hémisphère, et qui s'étend selon l'angle A.

La tête sphérique 2 peut être en céramique ou en métal. On pourra préférer le métal, pour des questions de coût. Il peut s'agir d'acier inoxydable ou de titane par exemple. Il en est de même de la cupule d'insertion 3.

La tête sphérique 2 comporte un logement tronconique 2c, apte à recevoir en force une extrémité de col de la première partie de prothèse de hanche.

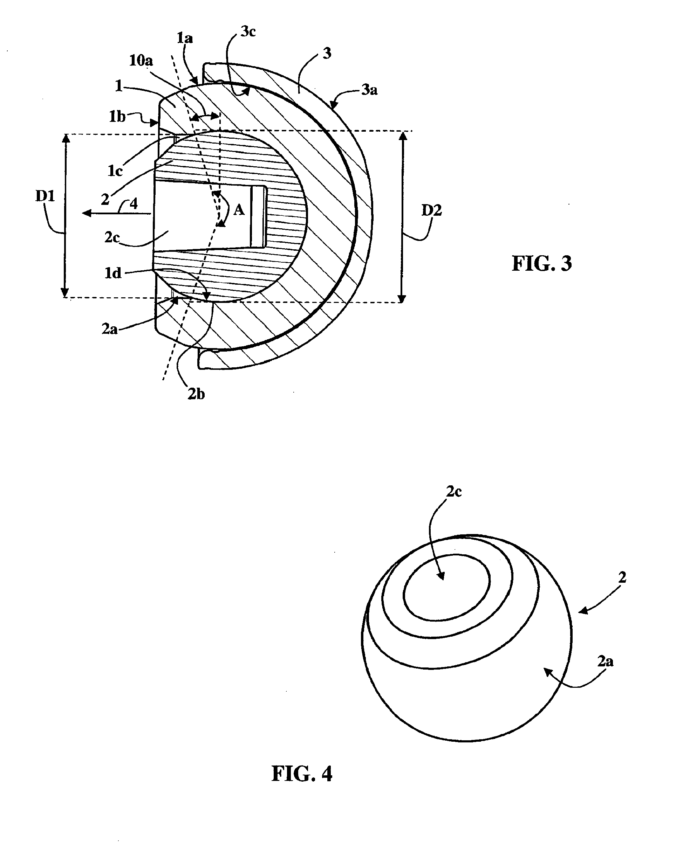

Sur les figures 4 à 6, on distingue plus clairement la structure de tête cylindrique 2, isolée des autres composants du cotyle. Les mêmes éléments sont repérés par les mêmes références numériques que sur les figures 1 à 3.

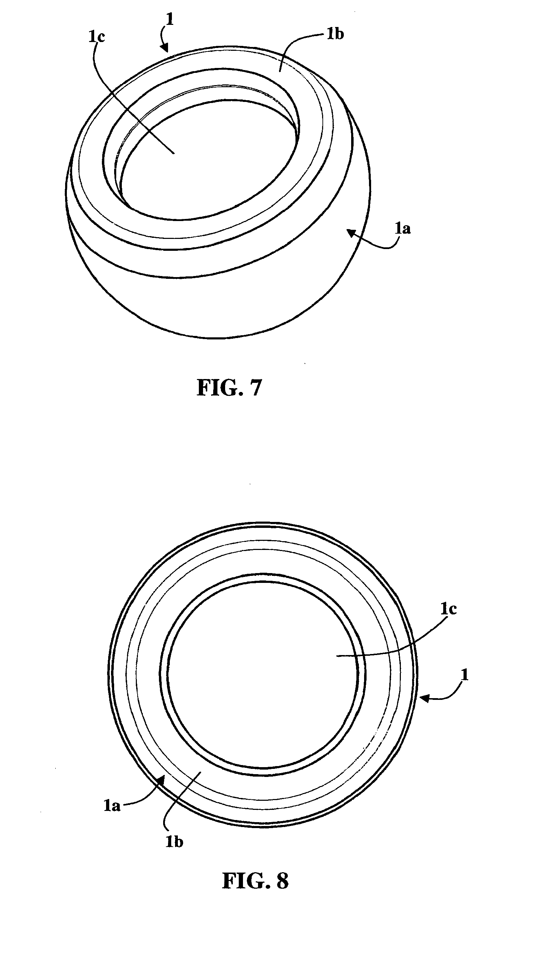

Sur les figures 7 à 9, on distingue plus clairement la structure de l'insert articulaire 1, qui est isolé des autres éléments du cotyle. Les mêmes éléments sont repérés par les mêmes références numériques que sur les figures 1 à 3.

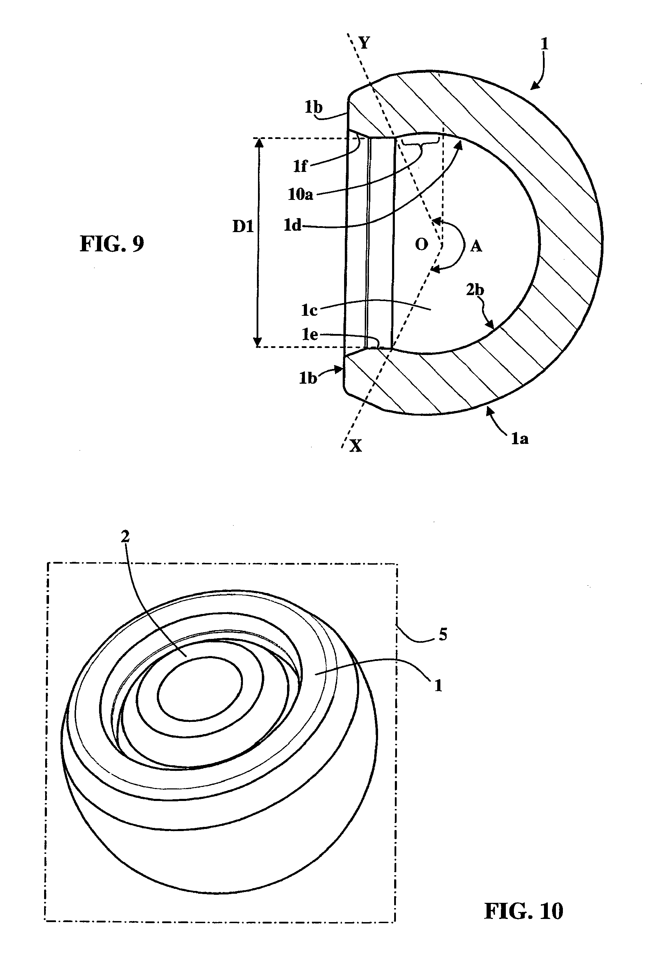

On distingue plus clairement, sur la figure 9, la surface articulaire 1d, et sa portion correspondant à la calotte sphérique 2b, limitée par un cône dont le sommet est au centre O de la tête sphérique 2 et de la surface articulaire 1d et qui est figuré par les lignes OX et OY. Cette calotte sphérique 2b est plus grande que la demi-sphère, et elle s'étend selon l'angle A ou angle selon lequel la tête sphérique 2 est engagée dans l'insert articulaire sur la figure 3. Au droit de l'intersection avec le cône OX-OY, la surface articulaire 1 d se poursuit par un tronçon cylindrique 1e de diamètre D1, puis par un bord chanfreiné qui se raccorde à la face réceptrice 1 b.

Selon l'invention, l'insert articulaire 1 et la tête sphérique 2 sont assemblés en usine puis stérilisés et conditionnés dans une enveloppe protectrice 5 comme illustré sur les figures 10 à 12. La cupule d'insertion 3 est conditionnée séparément.

Pour utiliser le cotyle, le praticien choisit une cupule d'insertion 3 qui convient à la morphologie de la cavité cotyloïdienne aménagée du patient à traiter, choisit un ensemble insert articulaire 1-tête sphérique 2 dont le logement 2c convient pour le réglage de longueur de première partie de prothèse en fonction des particularités anatomiques du patient à traiter, pose la cupule d'insertion 3 dans la cavité cotyloïdienne aménagée du bassin du patient, et assemble les autres éléments de la prothèse de hanche sans avoir à exécuter l'opération préalable d'assemblage en force de la tête sphérique 2 dans l'insert articulaire 1.

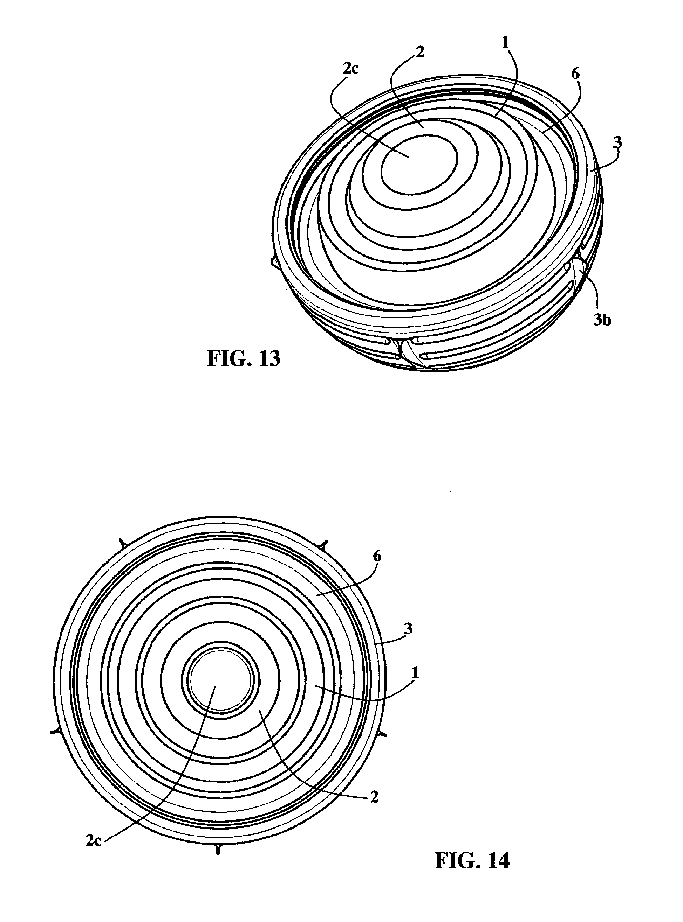

On considère maintenant un second mode de réalisation de cotyle prothétique de hanche selon l'invention tel qu'illustré sur les figures 13 à 15.

Dans ce mode de réalisation, on retrouve un insert articulaire 1 recevant et retenant une tête sphérique 2. L'insert articulaire 1 est engagé en rotation dans un insert intermédiaire 6 lui-même engagé de façon fixe dans une cupule d'insertion 3.

Dans ce mode de réalisation, la tête sphérique 2, l'insert articulaire 1 et l'insert intermédiaire 6 peuvent être en céramique. La cupule d'insertion 3 est en métal tel que l'acier inoxydable ou le titane, par exemple.

La tête sphérique 2 a la même forme que la tête 2 du mode de réalisation des figures 1 à 12.

L'insert articulaire 1 est similaire de celui du mode de réalisation précédent des figures 1 à 12, en ce qu'on retrouve la surface articulaire 1 d, la cavité articulaire 1c dans laquelle est engagée la tête sphérique 2, la face réceptrice 1b, et la surface externe sphérique 1 a.

La différence dans ce second mode de réalisation réside essentiellement dans la structure du moyen de rétention.

Dans ce cas, le moyen de rétention comprend une bague annulaire continue 10b, comportant une surface de retenue 10c en couronne sphérique apte à s'engager fonctionnellement avec un jeu 10d contre la tête sphérique 2, et comportant des excroissances périphériques 10e de fixation aptes à s'imbriquer fonctionnellement avec des retraits correspondants 1f de l'insert articulaire 1.

En pratique, la bague annulaire continue 10b est engagée dans un logement de forme correspondante prévu à l'entrée de la cavité articulaire 1c de l'insert articulaire 1. Les excroissances périphériques 10e comprennent par exemple une nervure périphérique de la bague annulaire continue 10b qui s'engage dans une rainure annulaire correspondante de l'insert articulaire 1. Lorsque la bague annulaire continue 10b est en place dans l'insert articulaire 1, sa surface de retenue 10c constitue un tronçon en contre-dépouille qui retient la tête sphérique 2 à l'intérieur de la cavité articulaire 1 c de l'insert articulaire 1.

Comme dans le mode de réalisation des figures 1 à 12, l'insert articulaire 1 et la tête sphérique 2 sont livrés assemblés, stériles, dans une enveloppe protectrice, avec la bague annulaire continue 10b en place, de sorte que, lors de l'utilisation de la prothèse pour sa pose, le praticien n'a pas à assembler en force la tête 2 dans l'insert articulaire 1.

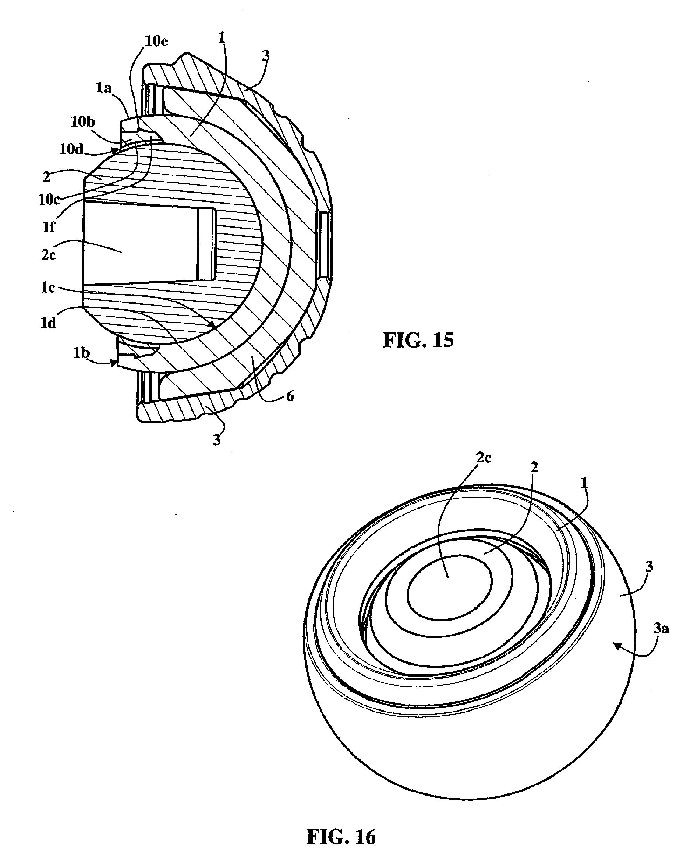

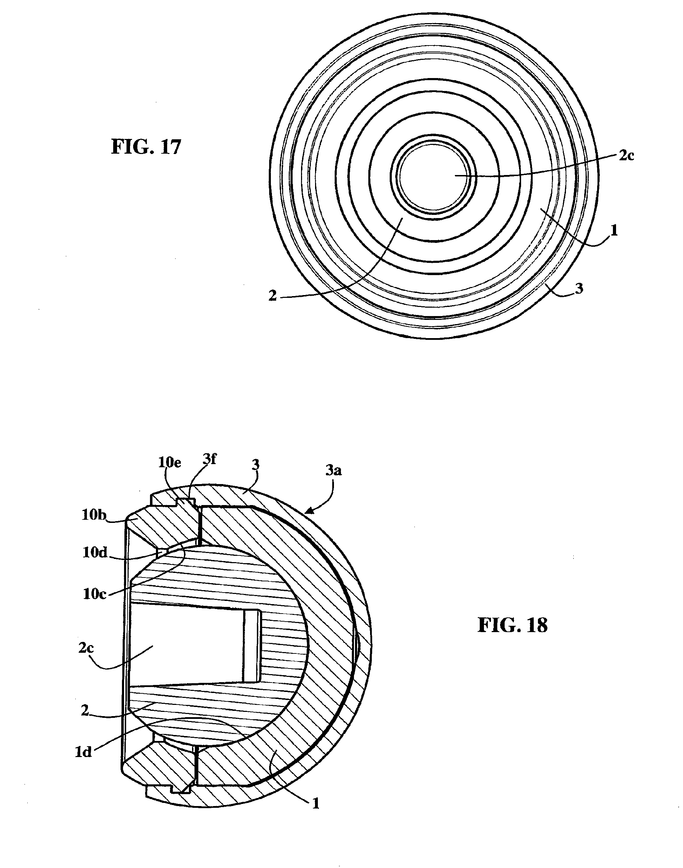

On considère maintenant un troisième mode de réalisation de cotyle prothétique de hanche selon l'invention, tel qu'illustré sur les figures 16 à 18. On retrouve une tête sphérique 2, identique à la tête du mode de réalisation précédent, on retrouve un insert articulaire 1, et on retrouve une cupule 3. Une première différence réside dans le fait que la cupule 3 est une cupule mobile dont la face extérieure 3a est sphérique et lisse pour constituer une surface de glissement apte à être engagée et pivotée dans la cavité cotyloïdienne d'un patient.

Dans ce mode de réalisation, la surface articulaire 1d de l'insert articulaire 1 est hémisphérique. L'insert articulaire 1 est en polyéthylène ou autre matière plastique équivalente, et est engagé de manière fixe dans la cupule 3. Le moyen de rétention est à nouveau une bague annulaire continue 10b, similaire de celle du mode de réalisation des figures 13 à 15, avec une surface de retenue 10c en couronne sphérique apte à s'engager fonctionnellement avec un jeu 10d contre la tête sphérique 2, et comportant des excroissances périphériques 10e de fixation.

Dans ce cas, les excroissances périphériques 10e s'imbriquent dans des retraits 3f ménagés dans la cavité de la cupule 3. Ainsi, la bague annulaire continue 10b est retenue par la cupule 3. Sa surface de retenue 10c en couronne sphérique constitue un tronçon en contre-dépouille de la surface articulaire 1d, qui s'oppose à l'extraction de la tête sphérique 2 à l'écart de l'insert articulaire 1.

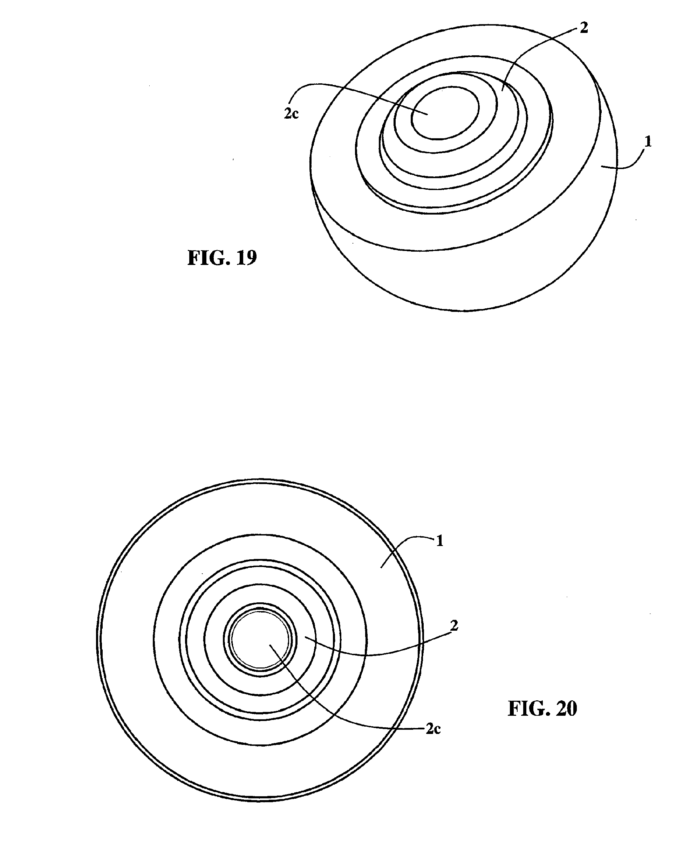

Les figures 19 à 21 illustrent un quatrième mode de réalisation de cotyle prothétique de hanche selon l'invention. On retrouve une tête sphérique 2 identique à celle des modes de réalisation précédents. L'insert articulaire 1 est en céramique, et comporte une surface articulaire 1d sensiblement hémisphérique. La surface externe 1a de l'insert articulaire 1 en céramique est sphérique, pour constituer une surface articulaire apte à pivoter dans une cavité cotyloïdienne du bassin d'un patient.

Dans ce mode de réalisation, le moyen de rétention est à nouveau constitué d'une bague annulaire continue 10b en polyéthylène ou autre matière plastique équivalente, ayant une surface de retenue 10c en couronne sphérique formant un tronçon en contre-dépouille de la surface articulaire 1 d, et avec des excroissances périphériques de fixation qui s'imbriquent fonctionnellement avec des retraits correspondants 1f de l'insert articulaire 1. On réalise ainsi un cotyle à cupule mobile en céramique.

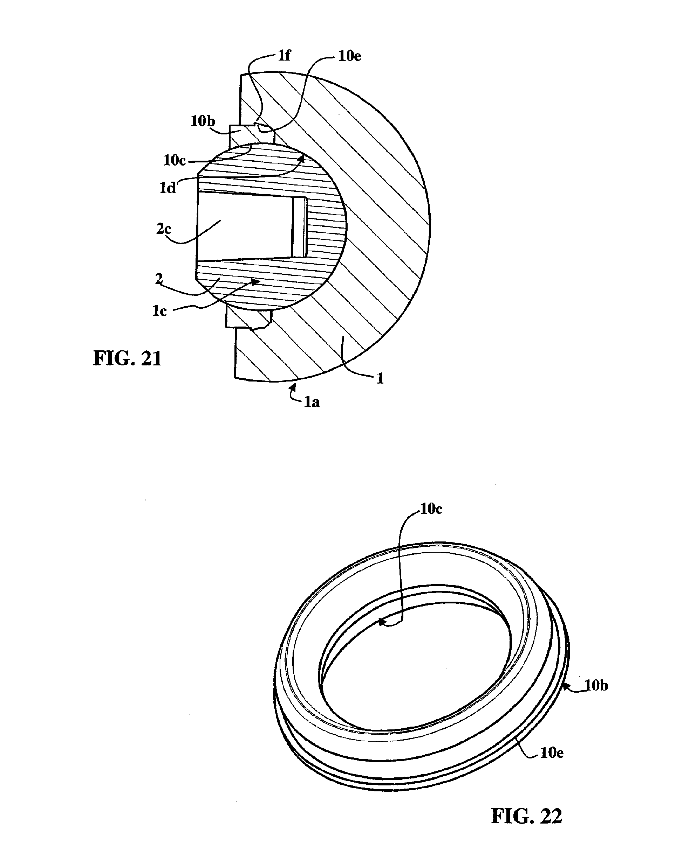

La figure 22 illustre en perspective le caractère continu de la bague annulaire 10b, telle que les bagues qui sont utilisées dans les modes de réalisation des figures 13 à 21.

Dans tous les modes de réalisation qui ont été décrits, le moyen de rétention est une pièce en polyéthylène ou en matière plastique équivalente, pièce qui doit être déformée lors de l'assemblage de la tête sphérique 2 dans l'insert articulaire 1. On peut par exemple utiliser le polyéthylène, le polyétheréthercétone (PEEK) ...

Dans le mode de réalisation des figures 1 à 3, il faut déformer l'embouchure de l'insert articulaire 1 pour la dilater afin de faciliter l'insertion de la tête sphérique 2.

Dans les autres modes de réalisation, il faut contracter la bague annulaire continue 10b pour faciliter son engagement dans l'insert articulaire 1 ou dans la cupule périphérique 3.

Pour cela, on soumet le moyen de rétention à une sollicitation thermique différentielle qui modifie temporairement ses dimensions pour faciliter son assemblage. Après assemblage, on laisse revenir le moyen de rétention à température ambiante, de sorte qu'il reprend sa forme d'origine et s'oppose alors à l'extraction de la tête sphérique 2 à l'écart de l'insert articulaire 1.

Dans le mode de réalisation des figures 1 à 3, dans lequel l'insert articulaire 1 constitue lui-même le moyen de rétention 10a et est réalisé en polyéthylène, la sollicitation thermique différentielle consiste à chauffer l'insert articulaire 1 pour augmenter temporairement son diamètre intérieur et faciliter ainsi l'introduction axiale de la tête sphérique 2.

Dans le cas d'un moyen de rétention sous forme d'une bague annulaire continue 10b, la sollicitation thermique différentielle consiste à refroidir la bague annulaire continue 10b pour réduire temporairement son diamètre extérieur et faciliter ainsi l'engagement des excroissances périphériques 10e de la bague dans les retraits correspondants 1f ou 3f respectifs de l'insert articulaire 1 ou de la cupule périphérique 3.

Selon l'invention, l'étape d'assemblage telle que définie ci-dessus est réalisée avant l'étape de stérilisation de l'ensemble tête sphérique 2-insert articulaire 1.

L'étape de stérilisation peut comprendre une étape de stérilisation par bombardement de rayons gamma.

On a pu constater, selon l'invention, que l'assemblage préalable avant stérilisation permet d'augmenter sensiblement la capacité de rétention de la tête sphérique dans l'insert articulaire, ce qui réduit les risques de luxation.

On a mis en évidence cet effet d'augmentation de la capacité de rétention en faisant des essais comparatifs de traction sur une tête engagée dans un insert articulaire, dans les conditions suivantes :
- tête en acier inoxydable de 28 mm ;
- insert articulaire en polyéthylène, ayant un tronçon de rétention en contre-dépouille de 4 mm de long, et ayant un diamètre extérieur de 48, 54 ou 62 mm ;
- force de traction axiale exercée entre la tête et l'insert articulaire.

Le tableau ci-dessous donne les valeurs de force limite provoquant la sortie de la tête sphérique hors de l'insert articulaire, pour les trois diamètres extérieurs d'insert articulaire, et dans chacun des deux cas : tête introduite dans l'insert articulaire après stérilisation, ou tête introduite dans l'insert articulaire avant stérilisation :

| Diamètre d'insert | Force produisant l'extraction d'une tête impactée après stérilisation (en daN) | Force produisant l'extraction d'une tête impactée avant stérilisation (en daN) |
|---|---|---|
| 48 | 42.71 | 50.10 |
| 54 | 54.01 | 65.32 |
| 62 | 65.21 | 73.03 |

La présente invention n'est pas limitée aux modes de réalisation qui ont été explicitement décrits, mais elle en inclut les diverses variantes et généralisations contenues dans le domaine des revendications ci-après.

## Revendications

1. Cotyle prothétique de hanche, comprenant :
- un insert articulaire (1), comportant une face réceptrice (1b) dans laquelle est ouverte une cavité (1c) à surface articulaire (1d) concave sensiblement hémisphérique,
- une tête sphérique (2) apte à être engagée dans la cavité articulaire (1c) de l'insert articulaire (1),
- un moyen de rétention (10a, 10b) pour s'opposer à la sortie axiale de la tête sphérique (2) hors de la cavité articulaire (1c) de l'insert articulaire (1) lorsque celle-ci est engagée dans la cavité articulaire (1c),
**caractérisé en ce que** :
- l'ensemble insert articulaire (1)-tête sphérique (2) est conditionné stérile dans une enveloppe protectrice (5),
- dans l'enveloppe protectrice (5), la tête sphérique (2) est engagée dans la cavité articulaire (1c) de l'insert articulaire (1) et est retenue par le moyen de rétention (10a, 10b).

2. Cotyle prothétique de hanche selon la revendication 1, **caractérisé en ce que** la force de rétention de la tête sphérique (2) exercée par le moyen de rétention (10a, 10b) est supérieure à un seuil de force déterminé.

3. Cotyle prothétique de hanche selon l'une des revendications 1 ou 2, **caractérisé en ce que**, dans la cavité articulaire (1c) et le moyen de rétention (10a, 10b), la tête sphérique (2) est engagée selon une calotte sphérique (2b) plus grande que l'hémisphère.

4. Cotyle prothétique de hanche selon la revendication 3, **caractérisé en ce que** la calotte sphérique (2b) s'étend selon un angle (A) supérieur à 190°.

5. Cotyle prothétique de hanche selon l'une quelconque des revendications 1 à 4, **caractérisé en ce que** le moyen de rétention (10a, 10b) est constitué par l'insert articulaire (1) lui-même, réalisé en polyéthylène ou autre matière plastique équivalente, et comportant un tronçon de rétention (10a) en contre-dépouille de surface articulaire (1d).

6. Cotyle prothétique de hanche selon la revendication 5, **caractérisé en ce que** l'insert articulaire (1) comporte une surface externe (1a) en calotte sphérique pour être monté rotatif dans une cupule d'insertion (3) et pour constituer ainsi un cotyle à double mobilité.

7. Cotyle prothétique de hanche selon l'une quelconque des revendications 1 à 4, **caractérisé en ce que** le moyen de rétention comprend une bague annulaire continue (10b), comportant une surface de retenue (10c) en couronne sphérique apte à s'engager fonctionnellement avec jeu (10d) contre la tête sphérique (2), et comportant des excroissances périphériques (10e) de fixation aptes à s'imbriquer fonctionnellement avec des retraits correspondants (1f) de l'insert articulaire (1) ou avec des retraits correspondants (3f) d'une cupule périphérique (3) entourant l'insert articulaire (1), pour interdire l'extraction de la bague annulaire continue (10b).

8. Cotyle prothétique de hanche selon la revendication 7, **caractérisé en ce que** la bague annulaire continue (10b) est en polyéthylène ou en une autre matière plastique équivalente.

9. Procédé d'assemblage pour réaliser un cotyle prothétique de hanche selon l'une quelconque des revendications 1 à 8, avec une tête sphérique (2) engagée dans une cavité (1 c) d'un insert articulaire (1) et retenue par un moyen de rétention (10a, 10b), le procédé ayant une étape de stérilisation des composants du cotyle et une étape d'assemblage de la tête sphérique (2) dans l'insert articulaire (1) et le moyen de rétention (10a, 10b), **caractérisé en ce que** l'on réalise l'étape de stérilisation après l'étape d'assemblage.

10. Procédé selon la revendication 9, **caractérisé en ce que**, au cours de l'étape d'assemblage, on soumet le moyen de rétention (10a, 10b) à une sollicitation thermique différentielle qui modifie temporairement ses dimensions pour faciliter l'assemblage, puis, après assemblage, on laisse le moyen de rétention reprendre la température ambiante pour qu'il s'oppose alors à l'extraction de la tête sphérique (2) à l'écart de l'insert articulaire (1).

11. Procédé selon la revendication 10, **caractérisé en ce que** :
- le moyen de rétention est l'insert articulaire (1) lui-même, réalisé en polyéthylène ou autre matière plastique équivalente,
- la sollicitation thermique différentielle consiste à chauffer l'insert articulaire (1) pour augmenter son diamètre intérieur et faciliter ainsi l'introduction axiale de la tête sphérique (2).

12. Procédé selon la revendication 10, **caractérisé en ce que** :
- le moyen de rétention est une bague annulaire continue (10b) en polyéthylène ou autre matière plastique équivalente,
- la sollicitation thermique différentielle consiste à refroidir la bague annulaire continue (10b) pour diminuer son diamètre intérieur et faciliter ainsi l'engagement des excroissances périphériques (10e) de la bague (10b) dans les retraits correspondants (1f, 3f) de l'insert articulaire (1) ou de la cupule périphérique (3).

## Patentansprüche

1. Prothetische Hüftgelenkspfanne mit:
- einem Gelenkeinsatz (1), der eine Aufnahmefläche (1b) aufweist, in der ein Hohlraum (1c) mit konkaver Gelenkfläche (1d) geöffnet ist, die im wesentlichen halbkugelförmig ist,
- einem sphärischen Kopf (2), der dazu ausgebildet ist, in den Gelenkhohlraum (1c) des Gelenkeinsatzes (1) einzugreifen,
- einem Rückhaltemittel (10a, 10b), um sich am axialen Ausgang des sphärischen Kopfes (2) außerhalb des Gelenkhohlraumes (1c) des Gelenkeinsatzes (1) abzustützen, wenn derselbe mit dem Gelenkhohlraum (1c) in Eingriff ist,
**dadurch gekennzeichnet, dass**:
- die Einheit Gelenkeinsatz (1) - sphärischer Kopf (2) in einer Schutzhülle (5) steril abgepackt ist,
- der sphärische Kopf (2) in der Schutzhülle (5) mit dem Gelenkhohlraum (1c) des Gelenkeinsatzes (1) in Eingriff ist und durch das Rückhaltemittel (10a, 10b) gehalten ist.

2. Prothetische Hüftgelenkspfanne nach Anspruch 1, **dadurch gekennzeichnet, dass** die Rückhaltekraft des sphärischen Kopfes (2), die durch das Rückhaltemittel (10a, 10b) aufgebracht ist, größer ist als ein vorbestimmter Schwellwert der Kraft.

3. Prothetische Hüftgelenkspfanne nach einem der Ansprüche 1 oder 2, **dadurch gekennzeichnet, dass** der sphärische Kopf (2) mit dem Gelenkhohlraum (1c) und dem Rückhaltemittel (10a, 10b) über eine sphärische Kappe (2b), die größer ist als die Kugel, in Eingriff ist.

4. Prothetische Hüftgelenkspfanne nach Anspruch 3, **dadurch gekennzeichnet, dass** sich die sphärische Kappe (2b) über einen Winkel (A) von mehr als 190° erstreckt.

5. Prothetische Hüftgelenkspfanne nach irgendeinem der Ansprüche 1-4, **dadurch gekennzeichnet, dass** das Rückhaltemittel (10a, 10b) durch den Gelenkeinsatz (1) selbst gebildet ist, aus Polyethylen oder anderem äquivalentem Kunststoffmaterial besteht und ein Rückhalteteil (10a) in Form einer Hinterschneidung zur Gelenkfläche (1d) enthält.

6. Prothetische Hüftgelenkspfanne nach Anspruch 5, **dadurch gekennzeichnet, dass** der Gelenkeinsatz (1) eine äußere Fläche (1a) in Form einer sphärischen Kappe aufweist, um drehbar in einem Einsatzbecher (3) befestigt zu werden und um auch eine Gelenkpfanne mit doppelter Beweglichkeit zu schaffen.

7. Prothetische Hüftgelenkspfanne nach irgendeinem der Ansprüche 1-4, **dadurch gekennzeichnet, dass** das Rückhaltemittel eine durchgehende ringförmige Buchse (10b) aufweist, die eine Rückhaltefläche (10c) mit sphärischem Kranz aufweist, die dazu ausgebildet ist, funktionell mit Spiel (10d) mit dem sphärischen Kopf (2) in Eingriff zu stehen, und periphere Befestigungsvorsprünge (10e) aufweist, die dazu ausgebildet sind, sich funktionell mit korrespondierenden Ausnehmungen (1f) des Gelenkeinsatzes (1) zu verkeilen, oder mit korrespondierenden Ausnehmungen (3f) einer peripheren Kappe (3), die den Gelenkeinsatz (1) umgibt, um ein Herausziehen der durchgehenden ringförmigen Buchse (10b) zu verhindern.

8. Prothetische Hüftgelenkspfanne nach Anspruch 7, **dadurch gekennzeichnet, dass** die umlaufende ringförmige Buchse (10b) aus Polyethylen oder einem anderen äquivalenten Kunststoffmaterial ist.

9. Montageverfahren zum Realisieren einer prothetischen Hüftgelenkspfanne nach irgendeinem der Ansprüche 1-8, mit einem sphärischen Kopf (2), der in einen Hohlraum (1c) eines Gelenkeinsatzes (1) eingreift und durch ein Rückhaltemittel (10a, 10b) zurückgehalten ist, wobei das Verfahren einen Schritt der Sterilisation der Bestandteile der Gelenkpfanne und einen Schritt der Montage des sphärischen Kopfes (2) in den Gelenkeinsatz (1) und in das Rückhaltemittel (10a, 10b) aufweist, **dadurch gekennzeichnet, dass** man den Schritt der Sterilisation nach dem Schritt der Montage durchführt.

10. Verfahren nach Anspruch 9, **dadurch gekennzeichnet, dass** man während des Schrittes der Montage das Rückhaltemittel (10a, 10b) einer unterschiedlichen thermischen Beanspruchung aussetzt, die temporär ihre Abmessungen zur Erleichterung der Montage modifiziert, anschließend, nach der Montage, das Rückhaltemittel die Umgebungstemperatur wieder einnehmen lässt, damit es sich bei einer Ausdehnung des sphärischen Kopfes (2) gegen den Gelenkeinsatz (1) abstützt.

11. Verfahren nach Anspruch 10, **dadurch gekennzeichnet, dass**:
- das Rückhaltemittel der Gelenkeinsatz (1) selbst ist, der aus Polyethylen oder anderem äquivalentem Kunststoffmaterial ist,
- die unterschiedliche thermische Beanspruchung darin besteht, den Gelenkeinsatz (1) zu erwärmen, um seinen inneren Durchmesser zu vergrößern und auch das axiale Einführen des sphärischen Kopfes (2) zu erleichtern.

12. Verfahren nach Anspruch 10, **dadurch gekennzeichnet, dass**:
- das Rückhaltemittel eine durchgehend ringförmige Buchse (10b) aus Polyethylen oder einem anderen äquivalenten Kunststoffmaterial ist,
- die unterschiedliche thermische Beanspruchung darin besteht, die umlaufende ringförmige Buchse (10b) zu kühlen, um ihren inneren Durchmesser zu verringern und auch den Eingriff der peripheren Vorsprünge (10e) der Buchse (10b) in die korrespondierenden Ausnehmungen (1f, 3f) des Gelenkeinsatzes (1) oder der peripheren Kappe (3) zu erleichtern.

## Claims

1. Prosthetic hip acetabulum, comprising:
- an articular insert (1), with a receiving face (1b) in which is opened a cavity (1c) with a substantially hemispherical, concave articular surface (1d),
- a spherical head (2), which can be engaged in the articular cavity (1c) of the articular insert (1),
- a retaining means (10a, 10b) for opposing the axial release of the spherical head (2) from the articular cavity (1c) of the articular insert (1) when it is engaged in the articular cavity (1c),
**characterized in that**:
- the assembly composed of articular insert (1) and spherical head (2) is packed in a sterile state in a protective envelope (5),
- in the protective envelope (5), the spherical head (2) is engaged in the articular cavity (1c) of the articular insert (1) and is retained by the retaining means (10a, 10b).

2. Prosthetic hip acetabulum as claimed in claim 1, **characterized in that** the force retaining the spherical head (2) and exerted by the retaining means (10a, 10b) is greater than a defined force threshold.

3. Prosthetic hip acetabulum as claimed in either of claims 1 or 2, **characterized in that**, in the articular cavity (1c) and the retaining means (10a, 10b), the spherical head (2) is engaged across a spherical cap (2b) larger than the hemisphere.

4. Prosthetic hip acetabulum as claimed in claim 3, **characterized in that** the spherical cap (2b) extends by an angle (A) greater than 190°.

5. Prosthetic hip acetabulum as claimed in any one of claims 1 through 4, **characterized in that** the retaining means (10a, 10b) is formed by the articular insert (1) itself, which is made of polyethylene or another equivalent plastic material and has a retaining segment (10a) undercut in the articular surface (1d).

6. Prosthetic hip acetabulum as claimed in claim 5, **characterized in that** the articular insert (1) has an outer surface (1a) in the shape of a spherical cap in order to be mounted rotatably in an insertion cup (3) and in order thereby to constitute a dual-mobility acetabulum.

7. Prosthetic hip acetabulum as claimed in any one of claims 1 through 4, **characterized in that** the retaining means comprises a continuous annular ring (10b), having a retaining surface (10c) in the shape of a spherical crown able to engage functionally with play (10d) against the spherical head (2), and having peripheral fixing projections (10e) able to interlock functionally with corresponding recesses (1f) of the articular insert (1) or with corresponding recesses (3f) of a peripheral cup (3) surrounding the articular insert (1), in order to prevent withdrawal of the continuous annular ring (10b).

8. Prosthetic hip acetabulum as claimed in claim 7, **characterized in that** the continuous annular ring (10b) is made of polyethylene or of another equivalent plastic material.

9. Method of assembly for producing a prosthetic hip acetabulum as claimed in any one of claims 1 through 8, with a spherical head (2) engaged in a cavity (1c) of an articular insert (1) and retained by a retaining means (10a, 10b), the method having a step of sterilizing the components of the acetabulum and an assembly step involving fitting the spherical head (2) in the articular insert (1) and the retaining means (10a, 10b), **characterized in that** the sterilizing step is performed after the assembly step.

10. Method as claimed in claim 9, **characterized in that**, during the assembly step, the retaining means (10a, 10b) is subjected to a differential thermal stress which temporarily modifies its dimensions in order to facilitate assembly, and then, after assembly, the retaining means is allowed to return to room temperature so as to then oppose the withdrawal of the spherical head (2) from the articular insert (1).

11. Method as claimed in claim 10, **characterized in that**:
- the retaining means is the articular insert (1) itself, which is made of polyethylene or another equivalent plastic material,
- the differential thermal stress involves heating the articular insert (1) in order to increase its internal diameter and thereby facilitate the axial introduction of the spherical head (2).

12. Method as claimed in claim 10, **characterized in that**:
- the retaining means is a continuous annular ring (10b) made of polyethylene or another equivalent plastic material,
- the differential thermal stress involves cooling the continuous annular ring (10b) in order to reduce its internal diameter and thereby facilitate the engagement of the peripheral projections (10e) of the ring (10b) in the corresponding recesses (1f, 3f) of the articular insert (1) or of the peripheral cup (3).
